(19) Europäisches Patentamt
European Patent Office
Office européen des brevets

(11) EP 3 718 602 A1

(12) **EUROPEAN PATENT APPLICATION**

(43) Date of publication:
**07.10.2020 Bulletin 2020/41**

(21) Application number: **19166734.4**

(22) Date of filing: **02.04.2019**

(51) Int Cl.:
*A61P 25/08* (2006.01) *A61K 31/19* (2006.01)
*A61K 31/53* (2006.01) *A61K 45/06* (2006.01)
*C07D 253/02* (2006.01) *A61K 9/14* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **University of Limerick**
**Limerick V94 T9PX (IE)**

(72) Inventors:
- **KAVANAGH, Oisín**
**Limerick, V94 T9PX (IE)**
- **LUSI, Matteo**
**Limerick, V94 T9PX (IE)**
- **WALKER, Gavin**
**Limerick, V94 T9PX (IE)**

(74) Representative: **Appleyard Lees IP LLP**
**15 Clare Road**
**Halifax HX1 2HY (GB)**

(54) **MULTICOMPONENT CRYSTAL FORMULATIONS**

(57) A multicomponent crystal (or co-crystal) comprising a first active pharmaceutical ingredient and a second active pharmaceutical ingredient. The multicomponent crystal is formed / sustained by non-covalent interactions between the nitrogen-containing heterocycle alpha-substituted with an amino group of the first active pharmaceutical ingredient and a carboxylic acid group of the second active pharmaceutical ingredient, suitably as well as other further non-covalent interactions with other H-bond forming groups. The multicomponent crystal may provide an improved multidrug dosage form comprising lamotrigine and valproic acid as the first and second active pharmaceutical ingredients, respectively. A pharmaceutical composition comprising a therapeutically effective amount of the multicomponent crystal and a pharmaceutically acceptable excipient, and a method of forming the multicomponent crystal, are also provided.

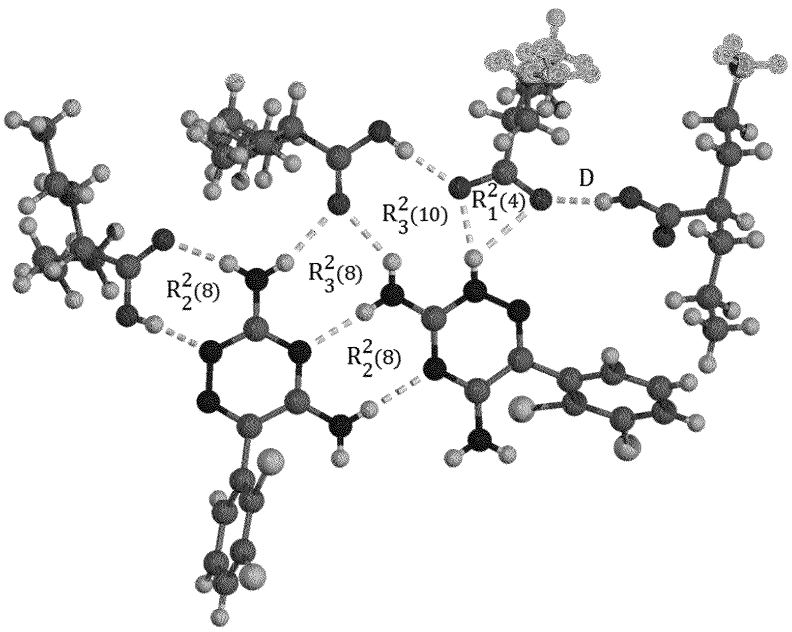

**Fig. 2**

EP 3 718 602 A1

## Description

### Field

The present invention relates to a multicomponent crystal (or co-crystal) comprising a first active pharmaceutical ingredient and a second active pharmaceutical ingredient, and to a pharmaceutical composition comprising a therapeutically effective amount of such a multicomponent crystal. In particular, the present invention relates to a multicomponent crystal comprising at least an interaction between a nitrogen-containing heterocycle substituted with an amino group of the first active pharmaceutical ingredient and a carboxylate group of the second active pharmaceutical ingredient, suitably further supported by additional H-bond interactions.

### Background

Many complex medical conditions require treatment with a combination of drugs, with each targeting a different aspect of the disease. However, complex drug regimens negatively affect patience compliance and result in poor adherence to therapy, which can lead to serious health consequences and increased costs to healthcare systems. In epilepsy, for instance, a single missed dose can lead to a fatal seizure. By enabling simpler drug regimens, multidrug dosage forms could provide safer and more effective therapeutic alternatives.

A multidrug dosage form can be obtained by co-formulating multiple active pharmaceutical ingredients (APIs) in the appropriate ratio to deliver the required effective amount of each API to the patient. However, only chemically compatible drug substances can be formulated together and the addition of excipients might be required to ensure adequate stability and bioavailability.

Therefore there is a need for multidrug dosage forms which have adequate or improved physical, pharmacokinetic and pharmacodynamic properties.

In epilepsy, adequate seizure control often requires combination therapy of lamotrigine and valproic acid. Lamotrigine is a solid class II drug (highly permeable and poorly soluble) according to the biopharmaceutical classification system (BCS). Orally it is 90% bioavailable but has a rather short half-life. Valproic acid is a liquid, which is predominantly marketed in its ionic co-crystal form as Depakote® or Epilim® (valproic acid and sodium valproate). In clinical use, the drug dosage is titrated according to seizure control. Therefore a multidrug dosage form comprising lamotrigine and valproic acid having good physical, pharmacokinetic and pharmacodynamic properties would be beneficial.

Co-crystals are solids that have a single crystalline single phase composed of two or more different molecular and/or ionic compounds. When one or both of the compounds in the co-crystal are ionic (i.e. salts), the resulting co-crystal is an ionic co-crystal. When both of the compounds of the co-crystal are neutral or zwitterionic molecules, the resulting co-crystal is a molecular co-crystal. A pharmaceutical co-crystal may be defined as a co-crystal which comprises an active pharmaceutical ingredient and a pharmaceutically acceptable compound (e.g. an excipient). The term "multicomponent crystal" may be used herein instead of "co-crystal" wherein at least one of the components of the "co-crystal" is in an ionic form.

### Summary of the Invention

It is one aim of the present invention, amongst others, to provide a multidrug dosage form that addresses at least one disadvantage of the prior art, whether identified here or elsewhere, or to provide an alternative to existing multidrug dosage forms. For instance it may be an aim of the present invention to provide a multicomponent crystal of a first API and a second API which may have improved drug properties compared to either of the first or second APIs alone.

According to aspects of the present invention, there is provided a multicomponent crystal and a pharmaceutical composition comprising a therapeutically effective amount and ratio of such components in a multicomponent crystal, as set forth in the appended claims. Other features of the invention will be apparent from the dependent claims, and the description which follows.

According to a first aspect of the present invention, there is provided a multicomponent crystal of a first active pharmaceutical ingredient and a second active pharmaceutical ingredient;

wherein the first active pharmaceutical ingredient comprises a nitrogen-containing heterocycle substituted with an amino group;

wherein the second active pharmaceutical ingredient comprises a carboxylic acid; and

wherein the nitrogen-containing heterocycle substituted with an amino group of the first active pharmaceutical ingredient interacts with the carboxylic acid of the second active pharmaceutical ingredient, in the multicomponent crystal.

The reference to the first and second active pharmaceutical ingredients is intended to mean that the first and second active pharmaceutical ingredients are different, i.e. different chemical entities, suitably having different pharmaceutical effects.

In the multicomponent crystal of this first aspect, the nitrogen-containing heterocycle substituted with an amino group of the first active pharmaceutical ingredient interacts with the carboxylic acid of the second active pharmaceutical ingredient. This interaction is suitably a non-covalent interaction between the stated functional groups, suitably a hydrogen bonding interaction. Suitably this interaction at least partially forms and sustains the multicomponent crystal structure.

Suitably the multicomponent crystal comprises further non-covalent interactions between the between the first and second active pharmaceutical ingredients,

suitably further hydrogen bonding interactions. Such additional or secondary interactions suitably also form and sustain the multicomponent crystal structure. Therefore the first active pharmaceutical ingredient and/or the second active pharmaceutical ingredient suitably form non-covalent interactions with other H-bond forming groups in the components of the multicomponent crystal, suitably H-bond interactions. Such other H-bond forming groups may be present in the first and/or second active pharmaceutical ingredients, or may be part of a third or further component in the multicomponent crystal, as discussed below.

Suitably the nitrogen-containing heterocycle of the first active pharmaceutical ingredient is alpha-substituted with the amino group. Therefore the amino group is suitably attached to a carbon atom adjacent to a nitrogen atom in the nitrogen-containing heterocycle.

The first and second active pharmaceutical ingredients may be present in the multicomponent crystal either in neutral or ionised form (i.e. the first active pharmaceutical ingredient may be protonated and the second active pharmaceutical ingredient may be deprotonated). The multicomponent crystal may comprise either or both of the neutral or ionised forms of either or both of the first and second active pharmaceutical ingredients.

The multicomponent crystal of this first aspect may be alternatively or additionally defined as a co-crystal of the stated components, insofar as that term can be used to cover such crystals comprising at least some ionic forms of the stated components.

The multicomponent crystal of this first aspect may be considered to be a crystalline composition comprising the stated multicomponent crystal form, suitably consisting essentially or, or consisting of, the stated multicomponent crystal form. The multicomponent crystal of this first aspect comprises a first API and a second API and therefore may be considered to be a multidrug dosage form consisting of such a multicomponent crystal. The multicomponent crystal of this first aspect may be additionally or alternatively referred to as a combination active pharmaceutical ingredient, which may be further formulated with suitable pharmaceutically acceptable excipients to provide a pharmaceutical composition for use as a medicament.

The inventors have surprisingly found that APIs having the stated functional groups can advantageously form a multicomponent crystal, comprising ionised components which, when sustained by H-bond interactions, may provide advantageous drug properties such as improved solubility, tablet-ability, thermal stability and chemical stability.

The multicomponent crystal of this first aspect comprises the first and second APIs. Suitably the multicomponent crystal consists essentially or consists of the first and second APIs. Suitably the first active pharmaceutical ingredient and the second active pharmaceutical ingredient are organic compounds. Suitably the multicomponent crystal comprises an ionic form or both an ionic and a neutral form of the first active pharmaceutical ingredient and an ionic form or both an ionic and a neutral form of the second active pharmaceutical ingredient.

In the multicomponent crystal of this first aspect, the interaction of the nitrogen-containing heterocycle substituted with an amino group of the first active pharmaceutical ingredient and the carboxylic acid group of the second active pharmaceutical ingredient suitably comprises an $R_1^2(4)$ or $R_2^2(8)$ motif. In some embodiments, the nitrogen-containing heterocycle of the first active pharmaceutical ingredient is protonated on the nitrogen in the alpha position with respect to the amino group and this proton interacts with the carboxylate group of the second active pharmaceutical ingredient, forming a $R_1^2(4)$ motif. Alternatively the nitrogen-containing heterocycle of the first active pharmaceutical ingredient is protonated in the alpha position with respect to the amino group and both protons of the amino group and N-substituted aromatic ring interact with the carboxylate group of the second active pharmaceutical ingredient to form an $R_2^2(8)$ motif. In both cases the $R_1^2(4)$ and $R_2^2(8)$ motif may be supported by further H-bond forming groups interacting with the carboxylate group.

Such further H-bond forming groups may be provided by the first and or second active pharmaceutical ingredients, or by additional molecules of the first and or second active pharmaceutical ingredients in the multicomponent crystal unit cell.

In some embodiments such further H-bond forming groups may be provided by a third component of the multicomponent crystal. The third component may be a third active pharmaceutical ingredient or it may be an inactive pharmaceutically acceptable excipient. The third component may be selected from water, carboxylic acids (acetic acid, NSAIDS), amines (pregablin, gabapentin or benzodiazepines), amides (peptides or caffeine), alcohols (methanol, ethanol, propanol or butanol), phenols (serotonin, adrenaline, vanillin or salicylic acids), mineral acids (hydrochloric, hydrobromic, phosphoric, sulfuric acid), imidazoles (fluconazole, itraconazole or metronidazole) or pyrroles (sunitinib, atorvastatin or Protoporphyrin IX).

Suitably the first API is any compound having a nitrogen-containing heterocycle substituted, preferably alpha-substituted, with an amino group which is pharmaceutically active. For example, the first API may be selected from lamotrigine, 4-aminopyridine, cytosine, thymine, 5-fluorocytosine, dihydralazine, endralazine, hydralazine, pipofezine, minaprine, cadralazine or cefozopran.

Suitably the second API is any compound having a carboxylic acid or a carboxylate group which is pharmaceutically active. For example the second API may be selected from valproic acid and/or a valproate salt, NSAIDs - including salicylate derivative NSAIDs, p-amino phenol derivative NSAIDs, propionic acid derivative NSAIDs, acetic acid derivative NSAIDs, enolic acid derivative NSAIDs and fenamic acid derivative NSAIDs - non-selective cyclo-oxygenase (cox) inhibitors, selective cyclooxygenase 1 (cox 1) inhibitors, selective cyclooxygenase 2 (cox 2) inhibitors or an antibiotic such as oxacillin, ampicillin, amoxicillin, cephalexin, cephalotin, cephalosporin, p-amino-salicylic acid, ciprofloxacin, enrofloxacin, difloxacin or danofloxacin.

Suitably the nitrogen-containing heterocycle substituted with an amino group of the first API has the structure (I):

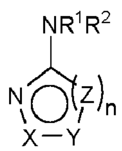

(I);

wherein $R^1$ and $R^2$ are each independently selected from H, a $C_1$-$C_8$ alkyl, a $C_1$-$C_8$ alkenyl, an aryl group, an alkylaryl group, a heteroaryl group or an alkylheteroaryl group, said groups optionally substituted with one or more of $C_1$-$C_4$ alkoxy, hydroxy, amino, carboxylic acid, ester, amide, halogen, $CF_3$, $CHF_2$ or $CH_2F$ groups;

wherein n = 0, 1, 2 or 3;

wherein X, Y and each Z are independently selected from N or C atoms;

wherein said N atoms are optionally substituted with a $C_1$-$C_8$ alkyl, a $C_1$-$C_8$ alkenyl, an aryl group, an alkylaryl group, a heteroaryl group or an alkylheteroaryl group, said groups optionally substituted with one or more of $C_1$-$C_4$ alkoxy, halogen, $CF_3$, $CHF_2$ or $CH_2F$ groups; and

wherein said C atoms are optionally substituted with $C_1$-$C_8$ alkyl, a $C_1$-$C_8$ alkenyl, an aryl group, an alkylaryl group, a heteroaryl group or an alkylheteroaryl group, optionally substituted with one or more of $C_1$-$C_4$ alkoxy, hydroxy, amino, carboxylic acid, ester, amide, halogen, $CF_3$, $CHF_2$ or $CH_2F$ groups, or wherein said C atoms are optionally substituted with $NR^5R^6$, wherein $R^5$ and $R^6$ are each independently selected from H, a $C_1$-$C_4$ alkyl, a $C_1$-$C_4$ alkenyl, an aryl group, an alkylaryl group, a heteroaryl group or an alkylheteroaryl group, said groups optionally substituted with one or more of $C_1$-$C_4$ alkoxy, hydroxy, amino, carboxylic acid, ester, amide, halogen, $CF_3$, $CHF_2$ or $CH_2F$ groups.

Suitably the first API has this structure (I).

Suitably at least one of X, Y and Z is a nitrogen atom. Suitably n = 1 or 2.

Suitably at least one of $R^1$ and $R^2$ are H. Suitably both $R^1$ and $R^2$ are H.

Suitably when any of X, Y and each Z are N atoms, said N atoms are unsubstituted, suitably apart from any H atom present to complete the required valency of the N atom in the heterocycle.

Suitably the nitrogen-containing heterocycle substituted with an amino group of the first API has the structure (II):

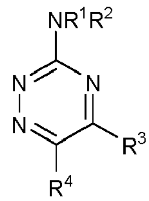

(II);

wherein $R^1$ and $R^2$ are each independently selected from H, a $C_1$-$C_8$ alkyl, a $C_1$-$C_8$ alkenyl, an aryl group, an alkylaryl group, a heteroaryl group or an alkylheteroaryl group, said groups optionally substituted with one or more of $C_1$-$C_4$ alkoxy, hydroxy, amino, carboxylic acid, ester, amide, halogen, $CF_3$, $CHF_2$ or $CH_2F$ groups; and

wherein $R^3$ and $R^4$ are each independently selected from H, $NR^5R^6$, $C_1$-$C_8$ alkyl, a $C_1$-$C_8$ alkenyl, an aryl group, an alkylaryl group, a heteroaryl group or an alkylheteroaryl group, said groups optionally substituted with one or more of $C_1$-$C_4$ alkoxy, hydroxy, amino, carboxylic acid, ester, amide, halogen, $CF_3$, $CHF_2$ or $CH_2F$ groups; wherein $R^5$ and $R^6$ are each independently selected from H, a $C_1$-$C_4$ alkyl, a $C_1$-$C_4$ alkenyl, an aryl group, an alkylaryl group, a heteroaryl group or an alkylheteroaryl group, said groups optionally substituted with one or more of $C_1$-$C_4$ alkoxy, hydroxy, amino, carboxylic acid, ester, amide, halogen, $CF_3$, $CHF_2$ or $CH_2F$ groups.

Suitably the nitrogen-containing heterocycle substituted with an amino group of the first API having the structure (I) or (II) comprises a further H-bond forming group, in addition to the amino group. For example, structure (I) or (II) may comprise a further amino group, e.g. $NR^5R^6$ as defined above, a carboxylic acid group, an amide group, an OH group or a suitable heterocyclic group.

Suitably the first API has this structure (II).

Suitably the nitrogen-containing heterocycle substituted with an amino group of the first API has the structure (III):

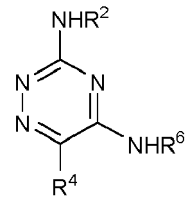

(III);

wherein $R^2$ is selected from H, a $C_1$-$C_4$ alkyl or a $C_1$-$C_4$ alkenyl, optionally substituted with one or more of $C_1$-$C_4$ alkoxy, hydroxy, amino, halogen, $CF_3$, $CHF_2$ or $CH_2F$ groups;

wherein $R^4$ is selected from H, $C_1$-$C_8$ alkyl, a $C_1$-$C_8$ alkenyl, an aryl group, an alkylaryl group, a heteroaryl group or an alkylheteroaryl group, said groups optionally substituted with one or more of $C_1$-$C_4$ alkoxy, hydroxy, amino, carboxylic acid, ester, amide, halogen, $CF_3$, $CHF_2$ or $CH_2F$ groups; and

wherein $R^6$ is selected from H, a $C_1$-$C_4$ alkyl, a $C_1$-$C_4$ alkenyl, an aryl group, an alkylaryl group, a heteroaryl group or an alkylheteroaryl group, said groups optionally substituted with one or more of $C_1$-$C_4$ alkoxy, hydroxy, amino, carboxylic acid, ester, amide, halogen, $CF_3$, $CHF_2$ or $CH_2F$ groups.

Suitably the first API has this structure (III).

Suitably $R^6$ is selected from H, a $C_1$-$C_4$ alkyl or a $C_1$-$C_4$ alkenyl, optionally substituted with one or more of $C_1$-$C_4$ alkoxy, hydroxy, amino, halogen, $CF_3$, $CHF_2$ or $CH_2F$ groups.

Suitably $R^2$ and $R^6$ are both H.

Suitably $R^4$ is an aryl group, optionally substituted with one or more of $C_1$-$C_4$ alkoxy, hydroxy, amino, halogen, $CF_3$, $CHF_2$ or $CH_2F$ groups.

Suitably the first active pharmaceutical ingredient is lamotrigine. Lamotrigine has the structure (IV):

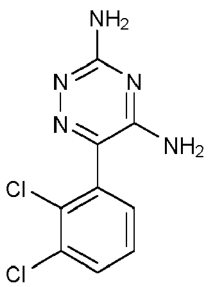

(IV).

Suitably the second API has the structure (V):

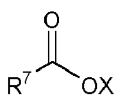

(V);

wherein X is H or a negative charge; and

wherein $R^7$ is selected from $C_1$-$C_{10}$ alkyl, a $C_1$-$C_{10}$ alkenyl, an aryl group, an alkylaryl group, a heteroaryl group or an alkylheteroaryl group, optionally substituted with one or more of $C_1$-$C_4$ alkoxy, halogen, $CF_3$, $CHF_2$ or $CH_2F$ groups.

When X is a negative charge, a suitable positively charged counterion is present in the multicomponent crystal; suitably a protonated and positively charged amino group on the first API.

Suitably $R^7$ is selected from $C_4$-$C_{10}$ alkyl or a $C_4$-$C_{10}$ alkenyl, optionally substituted with one or more of $C_1$-$C_4$ alkoxy, hydroxy, amino, carboxylic acid, ester, amide, halogen, $CF_3$, $CHF_2$ or $CH_2F$ groups. Suitably $R^7$ is a $C_4$-$C_8$ alkyl or a $C_4$-$C_8$ alkenyl, optionally substituted with one or more of $C_1$-$C_4$ alkoxy, halogen, $CF_3$, $CHF_2$ or $CH_2F$ groups.

Suitably the second active pharmaceutical ingredient is valproic acid and/or a valproate salt.

Valproic acid has the structure (VI):

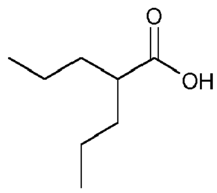

(VI).

Suitably the first API is lamotrigine and/or a lamotrigine salt and the second API is valproic acid and/or a valproate salt.

The inventors have found that such a multicomponent crystal comprising lamotrigine and valproic acid may provide an advantageous treatment for patients suffering from epilepsy, where lamotrigine and valproic acid are prescribed together. Such a multicomponent crystal may have improved tabletability and solubility compared to know forms of lamotrigine, which are particularly insoluble and difficult to form into tablets (lamotrigine is a solid class II drug - poorly soluble). Furthermore this multicomponent crystal may provide an alternative solid form of valproic acid which is a liquid in its neutral state under ambient conditions, and therefore may provide a more convenient dosage form of valproic acid.

Suitably the molar ratio of the first active pharmaceutical ingredient to the second active pharmaceutical ingredient in the multicomponent crystal is 1:2, suitably wherein the first API is lamotrigine and/or a lamotrigine salt and the second API is valproic acid and/or a valproate salt.

Patients being treated for epilepsy may receive a dual therapy comprising a maintenance dose of 1 g of valproic acid and a 100-200 mg dose of lamotrigine. Therefore a molar ratio of lamotrigine to valproic acid of approximately 1:9. However, due to pharmacodynamic and pharmacokinetic interactions, a 200 mg (0.781 mmol) does of lamotrigine may be used with a lower dose

of valproic acid (250 mg, 1.736 mmol). This provides a combined dose having a molar ratio of lamotrigine to valproic acid of approximately 1:2, which prolongs the circulation time of lamotrigine *in vivo*, and produces a synergistic effect. Therefore the multicomponent crystal of this first aspect comprising a 1:2 ratio of lamotrigine and valproic acid (and/or a valproate salt) may provide a particularly advantageous dosage form of this combined therapy for epilepsy which may have improved physical properties (e.g. tabletability and solubility), and may improve patient compliance due to the relative ease of taking a single combined dose of the two APIs.

Suitably the lamotrigine-valproic acid multicomponent crystal comprises an ionic form and a neutral form of the lamotrigine and an ionic form and a neutral form of the valproic acid.

According to a second aspect of the present invention, there is provided a multicomponent crystal according to the first aspect, for use as a medicament.

The multicomponent crystal for use as a medicament of this second aspect may have any of the suitable features and advantages described in relation to the first aspect. The multicomponent crystal may be used in the treatment of any disorder, which the first and second APIs are suitable for treating.

According to a third aspect of the present invention, there is provided a multicomponent crystal according to the first aspect, for use in the treatment of epilepsy. The multicomponent crystal of this third aspect may have any of the suitable features and advantages described in relation to the first aspect. Suitably the first API is lamotrigine and/or a lamotrigine salt and the second API is valproic acid and/or a valproate salt and the molar ratio of the lamotrigine and/or a lamotrigine salt to the valproic acid and/or a valproate salt in the multicomponent crystal is 1:2.

According to a fourth aspect of the present invention, there is provided a pharmaceutical composition comprising a therapeutically effective amount of a multicomponent crystal according to the first, second or third aspects, and a pharmaceutically acceptable excipient.

According to a fifth aspect of the present invention, there is provided a method of preparing a multicomponent crystal comprising at least two active pharmaceutical ingredients, the method comprising the steps of:

a) providing a first active pharmaceutical ingredient comprising a nitrogen-containing heterocycle substituted with an amino group;

b) providing a second active pharmaceutical ingredient comprising a carboxylic acid; and

c) combining the first active pharmaceutical ingredient and the second active pharmaceutical ingredient;

d) crystallising the combination of the first active pharmaceutical ingredient and the second active pharmaceutical ingredient obtained from step c) to provide the multicomponent crystal.

The multicomponent crystal formed by the method of this fifth aspect may have any of the suitable features or advantages described in relation to the first aspect.

Suitably the multicomponent crystal comprises an interaction between the nitrogen-containing heterocycle substituted with an amino group of the first active pharmaceutical ingredient and the carboxylic acid of the second active pharmaceutical ingredient.

Suitably step a) involves providing a powder of the first active pharmaceutical ingredient.

Suitably step b) involves providing a powder of the second active pharmaceutical ingredient.

Suitably step c) involves combining powders of the first active pharmaceutical ingredient and the second active pharmaceutical ingredient.

Step d) may involve forming a slurry of the first active pharmaceutical ingredient and the second active pharmaceutical ingredient and then drying to form a microcrystalline powder.

Suitably step d) involves, suitably after the formation of the slurry and drying, of dissolving in a solvent the first active pharmaceutical ingredient and the second active pharmaceutical ingredient and evaporating the solvent, to form the multicomponent crystal.

## Detailed Description Of The Example Embodiments

*Materials and methods*

In the following description, the comparative example lamotrigine (single API) is termed "LAM" and the multicomponent crystal of lamotrigine and valproic acid is termed "LAMVAL".

Lamotrigine and valproic acid (also termed "VAL" herein) were obtained from Baoji Guokang Bio-Technology Co. Ltd. and used without further purification. All other solvents and reagents were purchased from Sigma-Aldrich and used as received.

*Synthesis of lamotrigine:valproic acid (1:2) ionic cocrystal (LAMVAL):* 256.09 mg of lamotrigine (1 mmol) and 1.82 ml of valproic acid (2 mmol) were placed in a mortar and pestle and manually ground for 5 minutes until a dry, fine white powder was produced. Synthesis was also attempted by ball milling the same reagents in Retsch MM400 shaker mill in a 15 mL steel vessel with one 15 mm steel ball at 25 hz for 15 min.

Slurry experiments were conducted by stirring 256.09 mg of lamotrigine (1 mmol) and 1.82 ml of valproic acid (2 mmol) in deionised water (10 ml) for 48 h. The product was recovered by filtration and dried in air.

Recrystallization was attempted by slow evaporating a solution of about 5 mg of the microcrystalline powder in 20 ml of ethanol in a vial and by slow cooling

of a hot solution containing 5 mg of the microcrystalline powder in 20 ml of 1:1 isopropanol/methanol (*vide infra).*

*Powder X-ray diffraction (PXRD):* X-ray powder diffraction (XRPD) patterns were collected in Bragg-Brentano geometry on a PANalytical Empyrean diffractometer equipped with a sealed tube (Cu $K\alpha_{12}$, $\lambda$ = 1.5418 Å) an 1D X'Celerator detector between 4 and 40° $2\theta$.

Variable temperature PXRD data were collected in Bragg-Brentano geometry on a X'Pert MPD Pro equipped with a Anton-Paar TK450 stage, a sealed tube (Cu $K\alpha_{12}$, $\lambda$ = 1.5418 Å) and a 1D X'Celerator detector in the 4 - 30° ($2\theta$) range.

*Single-crystal X-ray Diffraction:* Single crystals were manually selected and mounted with Paratone® oil on a polymeric fibre. Data was collected at room temperature (298 K) as well as at 100 K on a Bruker Quest D8 diffractometer equipped with Mo sealed tube Tube (Mo-$K\alpha$ radiation $\lambda$ = 0.71073 Å), a Photon II CPAD detector and Oxford Cryosystem Cryostreem 800. Data was integrated with the APEX program suite and empirically corrected for absorption correction. Structure solution was found through direct methods in SHELX through X-Seed. All heavy atoms were found on the electron density map and refined anisotropically against all $F^2_{obb}$. H atoms were constrained through the riding model in their position as determined by an analysis of the distances between heavy atoms.

## Results and discussion

### *Crystal synthesis and structure analysis*

The neat grinding described above of a 1:2 ratio of LAM and VAL in a ball mill affords a dry microcrystalline powder. PXRD reveals that the product is stable as a slurry in water. Recrystallization by slow evaporation from ethanol resulted in crystals of a lamotrigine ethanolate (CSD ref. code GEVYOY). Recrystallization by slow cooling from isopropanol/methanol, affords quality single crystals of the title multicomponent crystal: LAMVAL. The monoclinic $P2_1/n$ unit cell comprises two LAM and four VAL independent residues. PXRD confirms the identity between the bulk powder and the single crystal, as shown in Figure 1 wherein the traces in order from the top show the PXRD data for lamotrigine; LAMVAL after stability test; LAMVAL after slurring in water; LAMVAL by ball mill; and calculated from LAMVAL single crystal data.

Since traditional X-ray diffraction cannot determine the hydrogen position in a reliable manner, the values of C-O distances were used to confirm that one LAM and one VAL species are ionised forming an ionic or salt multicomponent crystal. Crystals forms in which both components have mixed ionization state are rarely seen. LAMVAL may be considered to belong to a novel type of multicomponent crystal that could be represented as $(aA)A^+B^-(bB)$. Figure 2 shows a graph set analysis of LAMVAL highlighting key intermolecular interactions. The supramolecular structure is rather complex and multiple motifs can be recognised. One VAL molecule forms a typical $R^2_2(8)$ heterosynthon with the neutral LAM through the N2 donor. Another $R^2_2(8)$ motif forms between the aminopyridine moiety of the neutral LAM and the aminopyridine moiety of the cationic LAM involving both the N4 donors, as shown in Figure 2. Notably the triazine rings in the synthon lay on different planes. The carbonyl oxygen of a VAL stabilises the interaction by bridging the adjacent amine hydrogens: $R^2_3(8)$. The same VAL also donates into a charged assisted H-bond with the valproate generating a $R^2_3(10)$ motif. In the cation, the proton sits on the most basic nitrogen (N2) and a double, charge-assisted H-bond forms with the carboxylate anion: $R^2_1(4)$ motif. Finally, the last VAL donates into the valproate (D), and bridges with an adjacent complex to guarantee 1D H-bonded structure along the b crystallographic direction (Figure 2).

From a supramolecular point of view, the $R^2_1(4)$ motif is unusual. A CSD analysis revealed only another example of such motif (CSD ref. code VECVAD) out of 349 total entries containing aminopyridinium-carboxylate interactions (see methods). In all the other cases, the typical $R^2_2(8)$ motif is present, which involves both the pyridinium and the amino functions.

A CSD search of the multicomponent crystals that include LAM with carboxylic groups revealed that in 43 out of 48 cases LAM is protonated in the N2 position. In the remaining five cases (CSD ref. codes HUQIVA QIQHIJ QIQHOP WOKXUR GAVLEV), C-O bond distance analysis suggest that a partial charge transfer is present: the proton is reported either disordered over the nitrogen and oxygen positions or sitting half way. Such behaviour is consistent with the aforementioned idea of a continuum between neutral and ionised forms.

A visual inspection reveals that in all the structures with partial charge transfer, the $R^2_2(8)$ is always isolated. On the contrary, in the structures reported as salts, the carboxylate is always involved in at least another H-bond: with carboxylic, amine or hydroxyl donors. Indeed, a CSD search revealed other 62 cases of the same charge-assisted synthon involving either three or four functional groups in structures containing $\alpha$-aminopyridines, $\alpha$-aminopyrazine, or $\alpha$-aminotriazine with

carboxylic acids.

These observations suggest that the ionic character of the synthons could be related to the presence of ancillary H-bond donors. Such behaviour is exemplified by two forms of LAM and acetic acid (AA). LIBXUR is an ionic multicomponent crystal of LAM and AA in a 1:3 ratio that shows the same synthon as LAMVAL. In the new LAMAA 1:1 salt, two independent ionic $R_2^2(8)$ synthon are complemented by an H-bonded amine group each. In these cases, the difference in C-O distances 0.003(2), 0.001(2) and 0.045(2), suggest that the degree of ionization decreases with the H-bond donor distance: the 2.553(2), 2.752(2) and 2.912(2) respectively.

Such hypothesis was further confirmed computationally. The refinement of hydrogen positions in the four-molecular synthon of LIBXUR confirms that the ionic multicomponent crystal is the most stable form. On the contrary when the same refinement is performed on the isolated two-molecular $R_2^2(8)$ synthon, the multicomponent crystal form is the favoured (Figure 3). Figure 3 shows the following - Top: molecular and supramolecular distances as measured by single crystal XRD data for LIBXUR (left) and LAMAA (right). Bottom: molecular and supramolecular distances as measured from the DFT refined model LIBXUR (left) and LAMAA (right).

Fourier-transform infrared spectroscopy (FTIR) was employed to investigate the mechanism behind the solid state changes in the powder material. Figure 4 illustrates major peaks relating to presence of starting materials and demonstrated changes within the fingerprint region, suggestive of the formation of a new solid form. Of note, there is a shift of 1609 peak in LAM to 1638 in LAMVAL related to amine protons, a more pronounced peak at 1540 in LAMVAL, at 1444 slight peak change in LAMVAL, a new peak at 1058 in LAMVAL, 936 band missing in LAMVAL replaced by new band at 905 - 1005 and the increase of the 666 peak LAMVAL. All of these changes could be related to the formation of intermolecular H-bonds.

*Survey of Cambridge Structural Database (CSD):* The Cambridge Structural Database was searched through ConQuest (v.1.23, 2018) and the retrieved entries were subsequently analysed with Mercury (v.3.10.3, 2018). In all cases, the cut off distance for the interatomic interactions between H and O was set equal to the sum of vdW radii + 0.3 Å. This precaution was justified by the possible errors associated with the H position.

*Quantumechanical calculation:* Computational studies were performed with GAUSSIAN 09. Model structures were created starting from the crystallographic data replacing the propyl groups on each valproic acid with H atoms. The coordinates of all the H and O atoms plus those of selected C and N were refined by M06-2X/6-31+G(d,p) level of theory.

*Thermal Analysis:* Thermogravimetric analysis (TGA) was performed using a TA Instruments TGA-Q50 on at a constant rate of 10°C/min from 25°C to 350°C under a flux of nitrogen of 50 ml/min. Differential Scanning Calorimetry (DSC) was carried out using sealed aluminium pans on a TA instruments DSC-Q2000 differential scanning calorimeter. Temperature calibrations were made using indium as the standard. An empty pan, sealed in the same way as the sample, was used as a reference. All the thermograms were run at a heating/cooling rate of 10°C/min under a nitrogen purge at a rate of 50 ml/min. Figure 5 shows the TGA (top) and DSC (bottom) traces for LAMVAL.

*Scanning Electron Microscopy (SEM):* LAM and LAMVAL were separately placed onto carbon tape and coated with a thin layer of gold followed by analysis on a Joel CarryScope JCM-5700 scanning electron microscope. Micrographs were recorded at various magnifications using a beam voltage of 2.0 kV.

*Intrinsic solubility study:* Compacts of LAM and LAMVAL containing equivalent amounts of LAM were made by compacting 100 mg LAM and 220 mg of LAMVAL in an 8 mm punch and die set for 3 minutes using a hydraulic press with a compaction force of 5 tonnes. These compacts were each coated with paraffin wax, leaving one surface exposed and secured to the bottom of the dissolution apparatus with excess paraffin wax. Intrinsic solubility was determined using a 900 ml well filled with 900 ml of degassed, deionised water (or 0.1 M HCl solution) on a Pharma Test USP type II system. The solution had been previously equilibrated at 37°C and the paddle speed was set to 100 rpm after adding the samples. Aliquots of media were withdrawn at 5, 10, 15, 20 30 and 60 minutes (or 0.25, 0.5, 1, 3, 6, 12 and 24 hours), filtered through 0.45 μm PTFE filters and tested via a UV spectrometer in triplicate.

*Spectrometry:* Infrared analysis was performed on a Perkin-Elmer Spectrum 100 FT-IR spectrometer equipped with a solid-state ATR stage. UV-Vis absorbance measurements were carried on a Cary 60 UV-Vis spectrometer using 1 ml quartz cuvettes. Calibration curves were obtained by linear regression from a set of absorbance measurements from solutions on lamotrigine of known concentration preformed in triplicate.

*Tensile stress test:* Tensile strength analysis for lamotrigine and LAMVAL was conducted on tablets produced from 100 mg of milled powder (25 hz, 15 m) across a range of compaction forces using a 6 mm, flat-faced punch and die, using a Gamblen R-series tablet press. Hardness testing was performed immediately after tableting using a Pharma Test hardness tester. Each compaction force was tested in triplicate. Tensile strength was calculated according to the Equation 1 where F is the load required to fracture the tablet, D the table diameter and H the tablet height:

$$\text{Equation 1: Tensile strength} = 2F/\pi DH$$

*Stability testing:* Accelerated stability testing was conducted by taking 100 mg of powdered sample and placing it into a humidity chamber under 75% relative humidity at 40°C for 14 days. The samples were analysed before and after testing by PXRD, FT-IR, DSC and TGA.

*Physicochemical Characterization*

Thermogravimetric analysis shows that LAMVAL is thermally stable up to around 100°C. Differential scanning calorimetry and variable temperature PXRD reveals that the material undergoes an enantiotropic transition above 75°C (see Figure 6). Figure 6 shows the variable temperature PXRD for LAMVAL.

LAMVAL powder remains crystalline at 45°C at 75% relative humidity for two weeks (see Figure 1, second line from the top), and FT-IR and DSC (Figure 7) show no sign of product degradation. Figure 7 shows DSC (top) and FTIR (bottom) before and after accelerated stability testing.

*Pharmaceutical Characterization*

Scanning electron microscopy shows that a polycrystalline powder of LAMVAL is formed by clusters of smaller prisms whereas LAM forms large, smooth crystals (see Figure 8). Figure 8 shows SEM images of LAMVAL (left) and Lamotrigine (Right). It is well-known that crystal morphology can affect the mechanical properties of a material. The tensile strength was measured for the two materials.

Tablets of LAM and LAMVAL were prepared by compressing the polycrystalline powders at different pressure. Tablets of LAM obtained with a compaction weight lower than 400 kg were too brittle to enable hardness testing. Tablets of LAMVAL obtained in the same conditions demonstrate superior tableting properties with significantly greater tensile strength across a range of compaction forces (Figure 9). Figure 9 shows a comparison of physicochemical properties LAMVAL (circular data points) and pure Lamotrigine (square data points): top - tensile strength; middle - dissolution rate in PBS buffer (pH = 7.4); bottom - dissolution rate in 0.1 M HCl solution (pH = 1.2). In the bottom graph, the error bars are smaller than the data points.

Direct compression is the preferred method of tablet processing and enables this formulation to fit into continuous manufacturing processes, an area of particular interest for industry. Materials that show poor tabletting properties can be formulated with excipients that act as binders. Although effective, such procedures increase the size of the dosage form, a particular concern for multidrug formulations such as this. The improved mechanical properties of LAMVAL suggest that tablets could be manufactured with minimal use of excipients, which would increase the tablet size.

Measurements of intrinsic dissolution show that the ionic multicomponent crystal LAMVAL dissolves significantly (x2) faster than pure LAM (see Figure 9). In PBS buffer, at pH 7.4, the ionic multicomponent crystal affords a concentration of lamotrigine 20% higher than the pure base. In physiological acidic conditions (pH 1.2 M) LAMVAL affords a 33% increase in lamotrigine concentration.

Lamotrigine is a BCS class II drug, whose bioavailability is limited by poor solubility. Attempts at crystal engineering have been reported that aimed to find forms that are more soluble. In those cases, the formation of neutral adducts resulted in a material less soluble than LAM. On the contrary, LAMVAL produced a higher dissolution rate in *in vitro* physiological conditions, which could translate into increased bioavailability, without the need for excipients and therefore avoiding an increase in the size of the dosage form.

*Conclusions*

A stable ionic multicomponent crystal of LAM and VAL has been obtained either mechano-chemically or from solution in a reliable manner. The physicochemical properties such as dissolution rate and tabletability are significantly higher than those of LAM alone. Most importantly, the 1:2 stoichiometry appears to improve the pharmacokinetics of the APIs making LAMVAL an ideal candidate for a marketable multidrug dosage form.

The LAMVAL structure is sustained by a complex set of supramolecular motifs that include a four-component ionic synthon between the pyridinium a carboxylate and two ancillary carboxylic acids.

The present invention demonstrates that the use of high-order synthons can provide ionic multicomponent crystals between heterocycle-amine and carboxylic acids groups in different APIs to enable the preparation of multidrug dosage forms with improved drug properties.

In summary, the present invention provides a multicomponent crystal (or co-crystal) comprising a first active pharmaceutical ingredient and a second active pharmaceutical ingredient. The multicomponent crystal is formed / sustained by non-covalent interactions between the nitrogen-containing heterocycle alpha-substituted with an amino group of the first active pharmaceutical ingredient and a carboxylic acid group of the second active pharmaceutical ingredient, suitably as well as other further non-covalent interactions with other H-bond forming groups. The multicomponent crystal may provide an improved multidrug dosage form comprising lamotrigine and valproic acid as the first and second active pharmaceutical ingredients, respectively. A pharmaceutical composition comprising a therapeutically effective amount of the multicomponent crystal and a pharmaceutically acceptable excipient is also provided.

As used herein, the term "alkyl" means both

straight and branched chain saturated hydrocarbon groups. Examples of alkyl groups include methyl, ethyl, n-propyl, iso-propyl, n-butyl, t-butyl, i-butyl, and sec-butyl groups.

As used herein, the term "cycloalkyl" means a cyclic saturated hydrocarbon group. Examples of cycloalkyl groups include cyclopropyl, cyclobutyl, cyclopentyl and cyclohexyl.

As used herein, the term "aryl" means a carbocyclic aromatic system.

As used herein, the term "heteroaryl" means a cyclic aromatic system comprising at least one carbon atom and at least one heteroatom, for example at least one nitrogen atom.

As used herein, the term "halogen" or "halo" means fluorine, chlorine, bromine or iodine. Fluorine, chlorine and bromine are particularly preferred.

"Pharmaceutically acceptable salt" means a salt such as those described in standard texts on salt formation, see for example: P. Stahl, et a/., Handbook of Pharmaceutical Salts: Properties, Selection and Use (VCHA/WNey-VCH, 2002), or S.M. Berge, et a/., "Pharmaceutical Salts" (1977) Journal of Pharmaceutical Sciences, 66, 1-19. Suitable salts according to the invention include those formed with organic or inorganic acids or bases. In particular, suitable salts formed with acids according to the invention include those formed with mineral acids, strong organic carboxylic acids, such as alkanecarboxylic acids of 1 to 4 carbon atoms which are unsubstituted or substituted, for example, by halogen, such as saturated or unsaturated dicarboxylic acids, such as hydroxycarboxylic acids, such as amino acids, or with organic sulfonic acids, such as C,-C4alkyl- or aryl-sulfonic acids which are unsubstituted or substituted, for example by halogen. Pharmaceutically acceptable acid addition salts include those formed from hydrochloric, hydrobromic, sulphuric, nitric, citric, tartaric, acetic, phosphoric, lactic, pyruvic, acetic, trifluoroacetic, succinic, perchloric, fumaric, maleic, glycolic, lactic, salicylic, oxaloacetic, methanesulfonic, ethanesulfonic, p- toluenesulfonic, formic, benzoic, malonic, naphthalene-2-sulfonic, benzenesulfonic, isethionic, ascorbic, malic, phthalic, aspartic, and glutamic acids, lysine and arginine. Other acids, which may or may not in themselves be pharmaceutically acceptable, may be useful as intermediates in obtaining the compounds of the invention and their pharmaceutical acceptable acid addition salts.

Pharmaceutically acceptable base salts include ammonium salts, alkali metal salts, for example those of potassium and sodium, alkaline earth metal salts, for example those of calcium and magnesium, and salts with organic bases, for example dicyclohexylamine, N-methyl-D-glucomine, morpholine, thiomorpholine, piperidine, pyrrolidine, a mono-, di- or tri-lower alkylamine, for example ethyl-, tert-butyl-, diethyl-, diisopropyl-, triethyl-, tributyl- or dimethyl-propylamine, or a mono-, di- or trihydroxy lower alkylamine, for example mono-, di- or triethanolamine. Corresponding internal salts may furthermore be formed.

"Pharmaceutically acceptable solvate" means a molecular complex comprising the compound of the invention and one or more pharmaceutically acceptable solvent molecules, for example, water or ethanol. Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates, such as hydrates, exist when the drug substance incorporates solvent such as water, in the crystal lattice in either stoichiometric or non-stoichiometric amounts. Drug substances are routinely screened for the existence of hydrates since these may be encountered at any stage of the drug manufacturing process or upon storage of the drug substance or dosage form. Solvates are described in S. Byrn et a/., Pharmaceutical Research, 1995. 12(7): p. 954-954, and Water-Insoluble Drug Formulation, 2<nd>ed. R. Liu, CRC Press, page 553, which are incorporated herein by reference.

"Therapy", "treatment" and "treating" include both preventative and curative treatment of a condition, disease or disorder. It also includes slowing, interrupting, controlling or stopping the progression of a condition, disease or disorder. It also includes preventing, curing, slowing, interrupting, controlling or stopping the symptoms of a condition, disease or disorder.

Although a few preferred embodiments have been shown and described, it will be appreciated by those skilled in the art that various changes and modifications might be made without departing from the scope of the invention, as defined in the appended claims.

Throughout this specification, the term "comprising" or "comprises" means including the component(s) specified but not to the exclusion of the presence of other components. The term "consisting essentially of" or "consists essentially of" means including the components specified but excluding other components except for materials present as impurities, unavoidable materials present as a result of processes used to provide the components, and components added for a purpose other than achieving the technical effect of the invention. Typically, when referring to compositions, a composition consisting essentially of a set of components will comprise less than 5% by weight, typically less than 3% by weight, more typically less than 1 % by weight of non-specified components.

The term "consisting of" or "consists of" means including the components specified but excluding addition of other components.

Whenever appropriate, depending upon the context, the use of the term "comprises" or "comprising" may also be taken to encompass or include the meaning "consists essentially of" or "consisting essentially of", and may also be taken to include the meaning "consists of" or "consisting of".

The optional features set out herein may be used either individually or in combination with each other where appropriate and particularly in the combinations as set out in the accompanying claims. The optional features for each aspect or exemplary embodiment of the invention as set out herein are also to be read as applicable to any other aspect or exemplary embodiments of the invention, where appropriate. In other words, the skilled person reading this specification should consider the optional features for each exemplary embodiment of the invention as interchangeable and combinable between different exemplary embodiments.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, and drawings), and/or all of the steps of any method or process so disclosed, may be combined in any combination, except combinations where at least some of such features and/or steps are mutually exclusive.

Each feature disclosed in this specification (including any accompanying claims, and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, and drawings), or to any novel one, or any novel combination, of the steps of any method or process so disclosed.

## Claims

1. A multicomponent crystal of a first active pharmaceutical ingredient and a second active pharmaceutical ingredient;
wherein the first active pharmaceutical ingredient comprises a nitrogen-containing heterocycle substituted with an amino group;
wherein the second active pharmaceutical ingredient comprises a carboxylic acid group; and
wherein the nitrogen-containing heterocycle substituted with an amino group of the first active pharmaceutical ingredient interacts with the carboxylic acid of the second active pharmaceutical ingredient, in the multicomponent crystal.

2. The multicomponent crystal according to claim 1, wherein the first active pharmaceutical ingredient and/or the second active pharmaceutical ingredient form non-covalent interactions with other H-bond forming groups of the components of the multicomponent crystal.

3. The multicomponent crystal according to claim 1 or claim 2, wherein the nitrogen-containing heterocycle substituted with an amino group of the first active pharmaceutical ingredient has the structure (I):

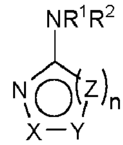

(I);

wherein $R^1$ and $R^2$ are each independently selected from H, a $C_1$-$C_8$ alkyl, a $C_1$-$C_8$ alkenyl, an aryl group, an alkylaryl group, a heteroaryl group or an alkylheteroaryl group, optionally substituted with one or more of $C_1$-$C_4$ alkoxy, hydroxy, amino,carboxylic acid, ester, amide, halogen, $CF_3$, $CHF_2$ or $CH_2F$ groups;
wherein n = 0, 1, 2 or 3;
wherein X, Y and each Z are independently selected from N or C atoms;
wherein said N atoms are optionally substituted with a $C_1$-$C_8$ alkyl, a $C_1$-$C_8$ alkenyl, an aryl group, an alkylaryl group, a heteroaryl group or an alkylheteroaryl group, which are optionally substituted with one or more of $C_1$-$C_4$ alkoxy, hydroxy, amino, carboxylic acid, ester, amide, halogen, $CF_3$, $CHF_2$ or $CH_2F$ groups; and
wherein said C atoms are optionally substituted with $C_1$-$C_8$ alkyl, a $C_1$-$C_8$ alkenyl, an aryl group, an alkylaryl group, a heteroaryl group or an alkylheteroaryl group, optionally substituted with one or more of $C_1$-$C_4$ alkoxy, hydroxy, amino, carboxylic acid, ester, amide, halogen, $CF_3$, $CHF_2$ or $CH_2F$ groups, or wherein said C atoms are optionally substituted with $NR^5R^6$, wherein $R^5$ and $R^6$ are each independently selected from H, a $C_1$-$C_4$ alkyl, a $C_1$-$C_4$ alkenyl, an aryl group, an alkylaryl group, a heteroaryl group or an alkylheteroaryl group, optionally substituted with one or more of $C_1$-$C_4$ alkoxy, hydroxy, amino, carboxylic acid, ester, amide, halogen, $CF_3$, $CHF_2$ or $CH_2F$ groups.

4. The multicomponent crystal according to claim 1 or claim 2, wherein the nitrogen-containing heterocycle substituted with an amino group of the first active pharmaceutical ingredient has the structure (II):

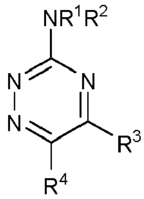

(II);

wherein $R^1$ and $R^2$ are each independently selected from H, a $C_1$-$C_8$ alkyl, a $C_1$-$C_8$ alkenyl, an aryl group, an alkylaryl group, a heteroaryl group or an alkylheteroaryl group, optionally substituted with one or more of $C_1$-$C_4$ alkoxy, hydroxy, amino, halogen, $CF_3$, $CHF_2$ or $CH_2F$ groups; wherein $R^3$ and $R^4$ are each independently selected from H, $NR^5R^6$, $C_1$-$C_8$ alkyl, a $C_1$-$C_8$ alkenyl, an aryl group, an alkylaryl group, a heteroaryl group or an alkylheteroaryl group, optionally substituted with one or more of $C_1$-$C_4$ alkoxy, hydroxy, amino, carboxylic acid, ester, amide, halogen, $CF_3$, $CHF_2$ or $CH_2F$ groups; wherein $R^5$ and $R^6$ are each independently selected from H, a $C_1$-$C_4$ alkyl, a $C_1$-$C_4$ alkenyl, an aryl group, an alkylaryl group, a heteroaryl group or an alkylheteroaryl group, optionally substituted with one or more of $C_1$-$C_4$ alkoxy, hydroxy, amino, carboxylic acid, ester, amide, halogen, $CF_3$, $CHF_2$ or $CH_2F$ groups.

**5.** The multicomponent crystal according to any one of the preceding claims, wherein the second active pharmaceutical ingredient has the structure (V):

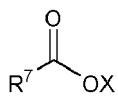

(V);

wherein X is H or a negative charge; and wherein $R^7$ is selected from $C_1$-$C_{10}$ alkyl, a $C_1$-$C_{10}$ alkenyl, an aryl group, an alkylaryl group, a heteroaryl group or an alkylheteroaryl group, optionally substituted with one or more of $C_1$-$C_4$ alkoxy, hydroxy, amino, carboxylic acid, ester, amide, halogen, $CF_3$, $CHF_2$ or $CH_2F$ groups.

**6.** The multicomponent crystal according to claim 1, wherein the first active pharmaceutical ingredient is selected from lamotrigine, 4-aminopyridine, cytosine, thymine, 5-fluorocytosine, dihydralazine, endralazine, hydralazine, pipofezine, minaprine, cadralazine or cefozopran.

**7.** The multicomponent crystal according to claim 1 or claim 6, wherein the second active pharmaceutical ingredient is selected from valproic acid and/or a valproate salt, NSAIDs - including salicylate derivative NSAIDs, p-amino phenol derivative NSAIDs, propionic acid derivative NSAIDs, acetic acid derivative NSAIDs, enolic acid derivative NSAIDs and fenamic acid derivative NSAIDs - non-selective cyclo-oxygenase (cox) inhibitors, selective cyclooxygenase 1 (cox 1) inhibitors, selective cyclooxygenase 2 (cox 2) inhibitors or an antibiotic such as oxacillin, ampicillin, amoxicillin, cephalexin, cephalotin, cephalosporin, p-amino-salicylic acid, ciprofloxacin, enrofloxacin, difloxacin or danofloxacin.

**8.** The multicomponent crystal according to claim 1, wherein the first active pharmaceutical ingredient is lamotrigine and wherein the second active pharmaceutical ingredient is valproic acid.

**9.** The multicomponent crystal according to any one of the preceding claims, wherein the molar ratio of the first active pharmaceutical ingredient to the second active pharmaceutical ingredient in the multicomponent crystal is 1:2.

**10.** The multicomponent crystal according to any one of the preceding claims, wherein the interaction of the nitrogen-containing heterocycle substituted with an amino group of the first active pharmaceutical ingredient and the carboxylic acid group of the second active pharmaceutical ingredient comprises an $R_1^2(4)$ synthon.

**11.** The multicomponent crystal according to any one of the preceding claims, comprising an ionic form and a neutral form of the first active pharmaceutical ingredient and an ionic form and a neutral form of the second active pharmaceutical ingredient; and wherein the first active pharmaceutical ingredient and the second active pharmaceutical ingredient are organic compounds.

**12.** The multicomponent crystal according to any one of claims 1 to 11, for use as a medicament.

**13.** The multicomponent crystal according to any one of claims 1 to 11, for use in the treatment of epilepsy.

**14.** A method of preparing a multicomponent crystal comprising at least two active pharmaceutical ingredients, the method comprising the steps of:

a) providing a first active pharmaceutical ingredient comprising a nitrogen-containing heterocycle substituted with an amino group;
b) providing a second active pharmaceutical ingredient comprising a carboxylic acid; and
c) combining the first active pharmaceutical ingredient and the second active pharmaceutical ingredient;

d) crystallising the combination of the first active pharmaceutical ingredient and the second active pharmaceutical ingredient obtained from step c) to provide the multicomponent crystal.

**15.** A pharmaceutical composition comprising a therapeutically effective amount of a multicomponent crystal according to any one of claims 1 to 12, and a pharmaceutically acceptable excipient.

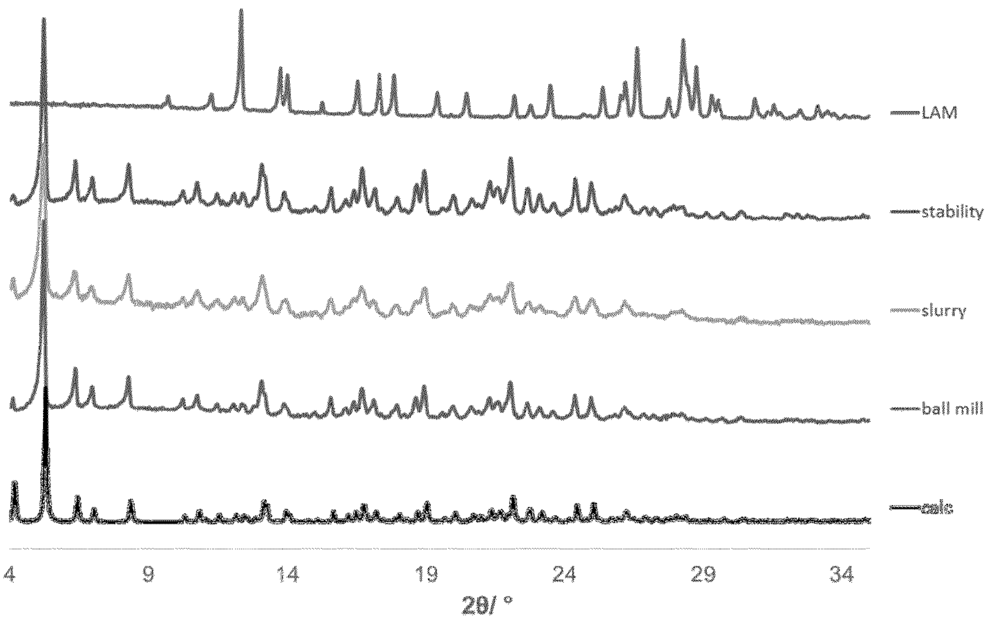
LAM
stability
slurry
ball mill
calc
4
9
14
19
24
29
34
2θ/ °

Fig. 1

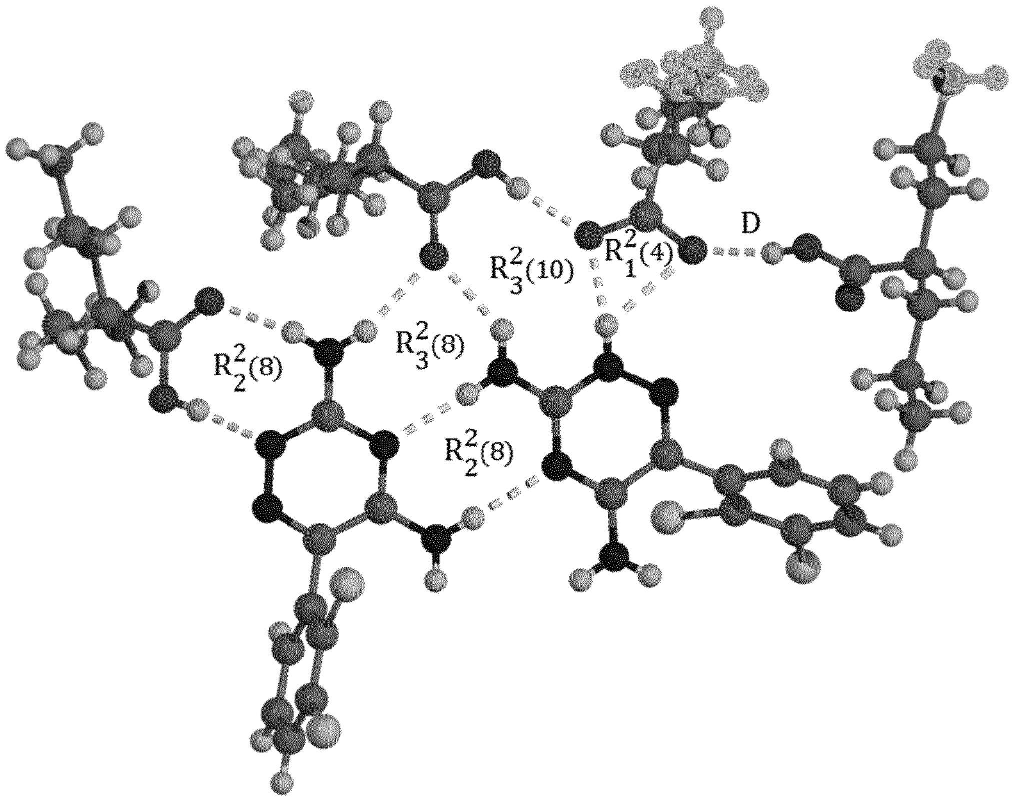
D
$R^2_1(4)$
$R^2_3(10)$
$R^2_2(8)$
$R^2_3(8)$
$R^2_2(8)$

Fig. 2

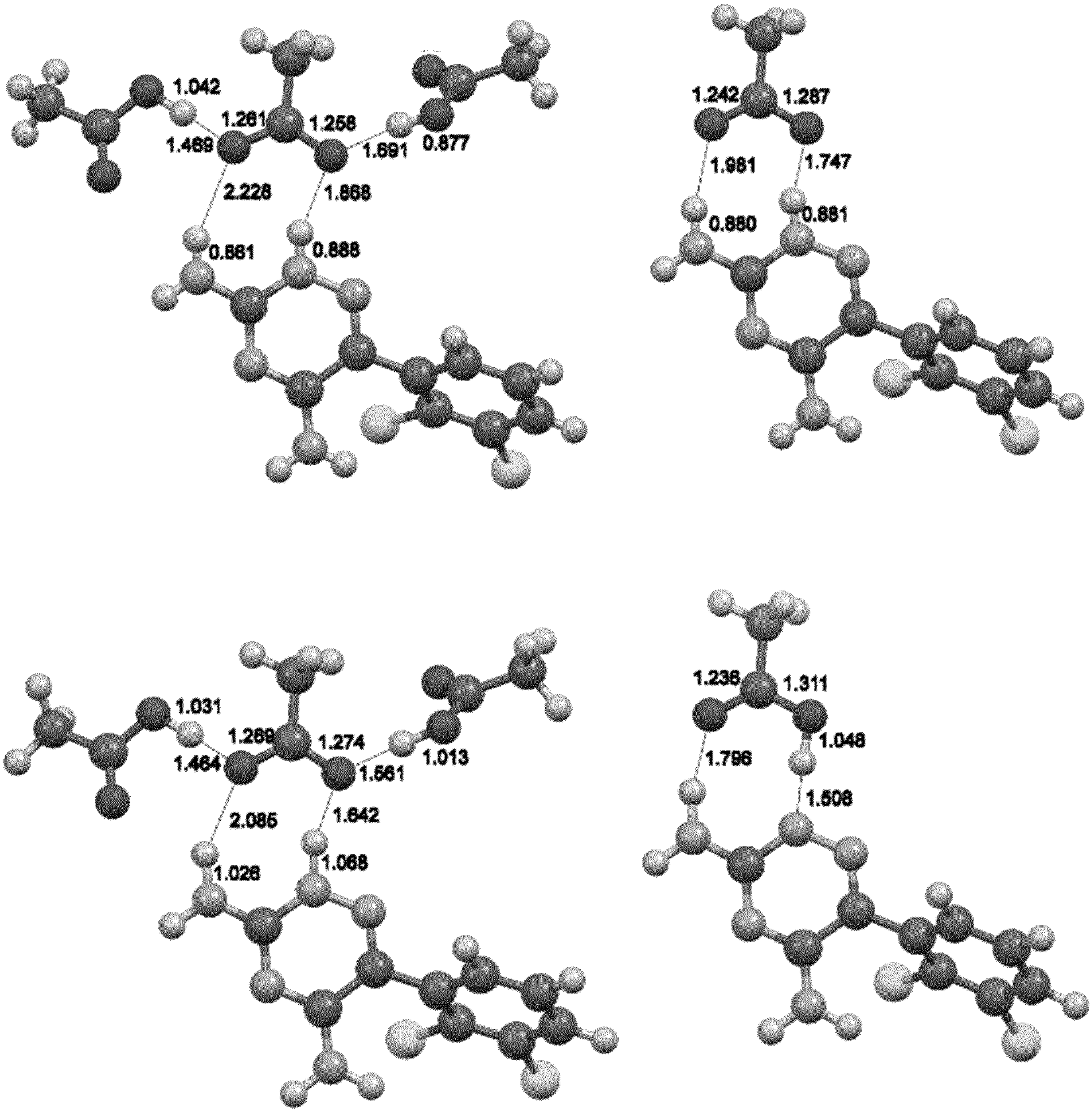
1.042 1.261 1.258 1.469 1.691 0.877 2.228 1.868 0.861 0.888
1.242 1.287 1.981 1.747 0.880 0.861
1.031 1.269 1.274 1.464 1.561 1.013 2.085 1.642 1.026 1.068
1.236 1.311 1.048 1.796 1.508

**Fig. 3**

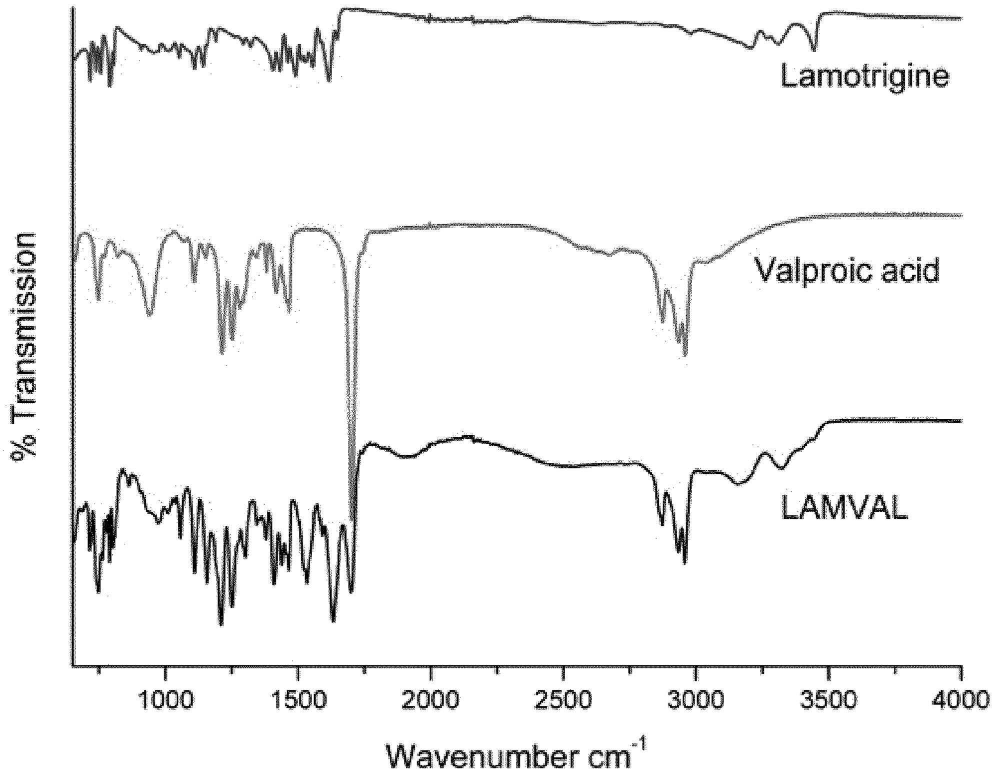
Lamotrigine
Valproic acid
LAMVAL
% Transmission
1000
1500
2000
2500
3000
3500
4000
Wavenumber cm-1

Fig. 4

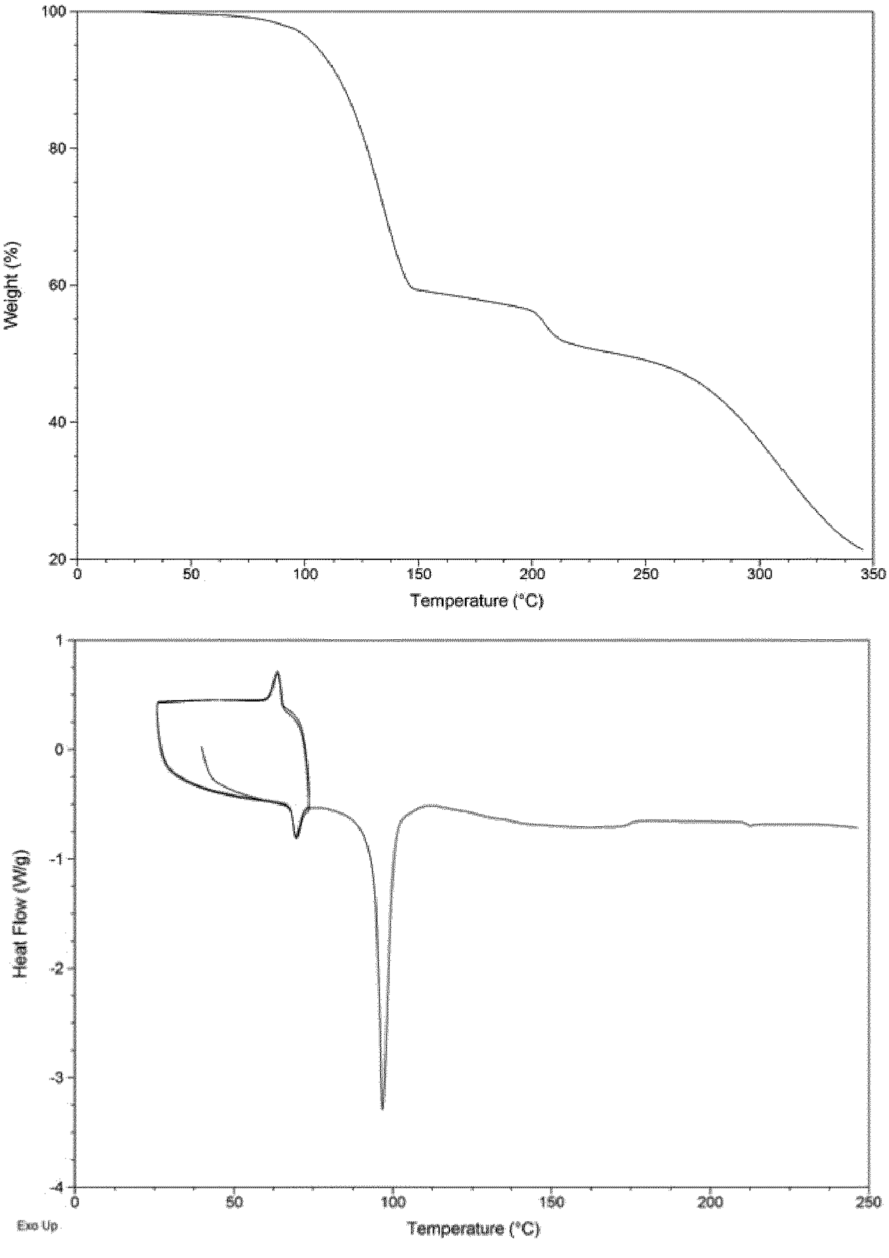
100
80
60
40
20
Weight (%)
0
50
100
150
200
250
300
350
Temperature (°C)
1
0
-1
-2
-3
-4
Heat Flow (W/g)
0
50
100
150
200
250
Exo Up
Temperature (°C)

**Fig. 5**

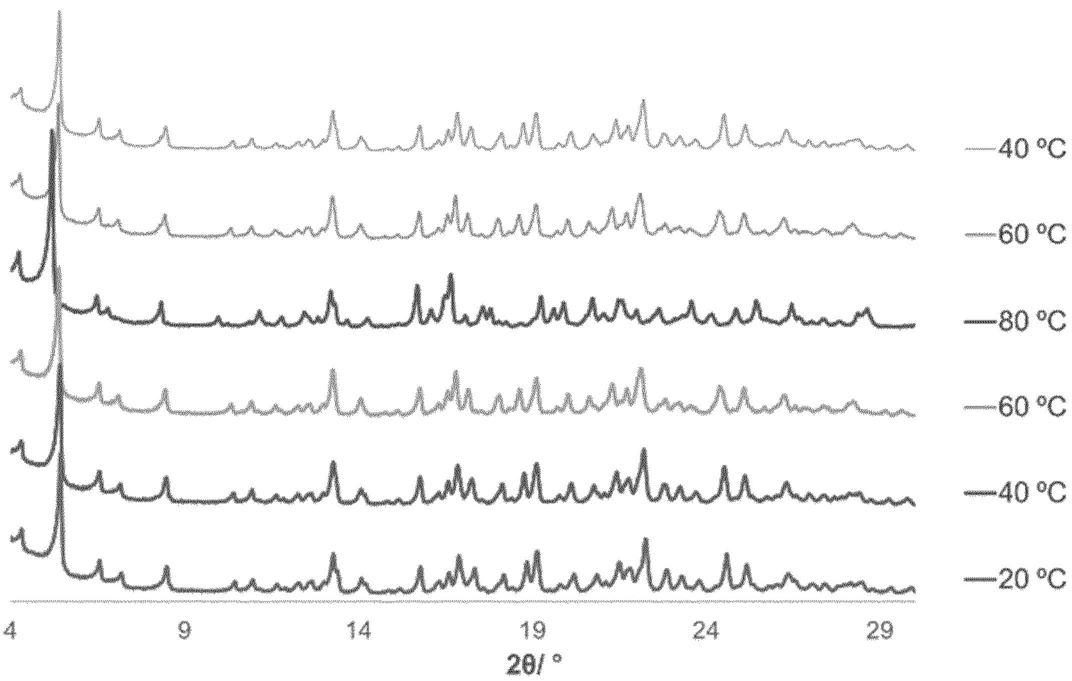


**Fig. 6**

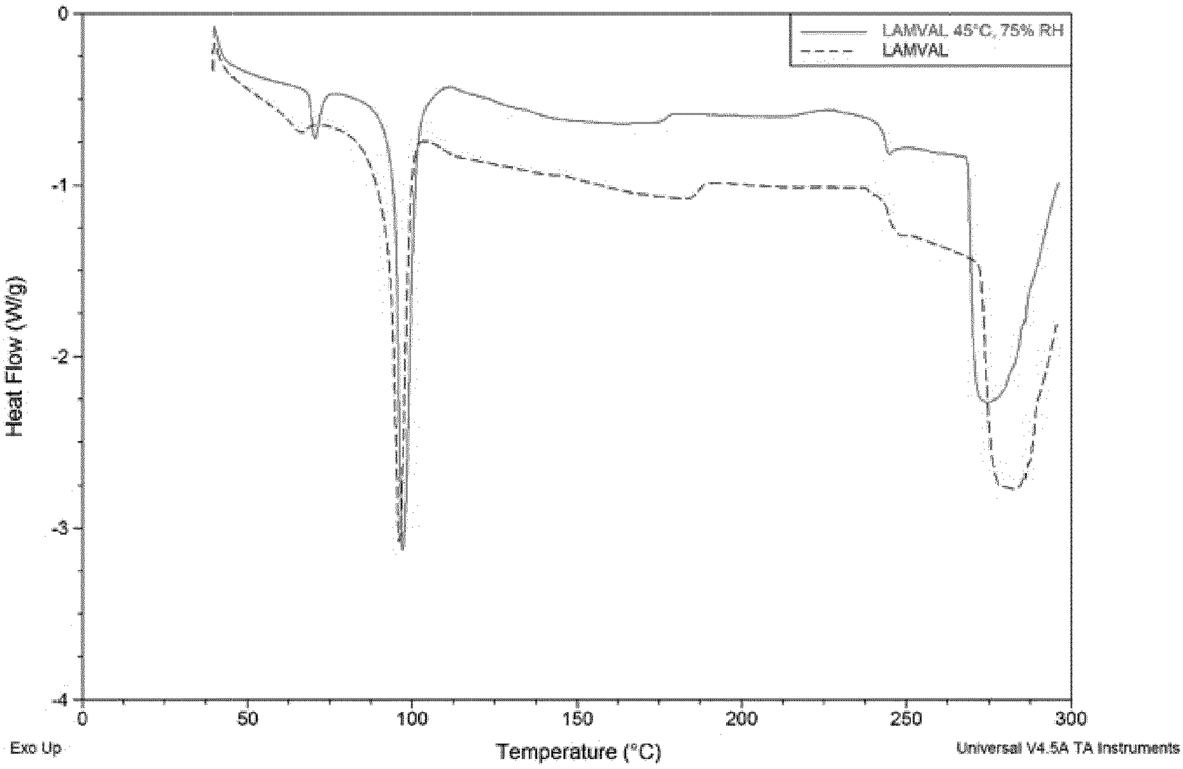
LAMVAL 45°C, 75% RH
LAMVAL
Heat Flow (W/g)
Temperature (°C)
Exo Up
Universal V4.5A TA Instruments
0
-1
-2
-3
-4
0
50
100
150
200
250
300

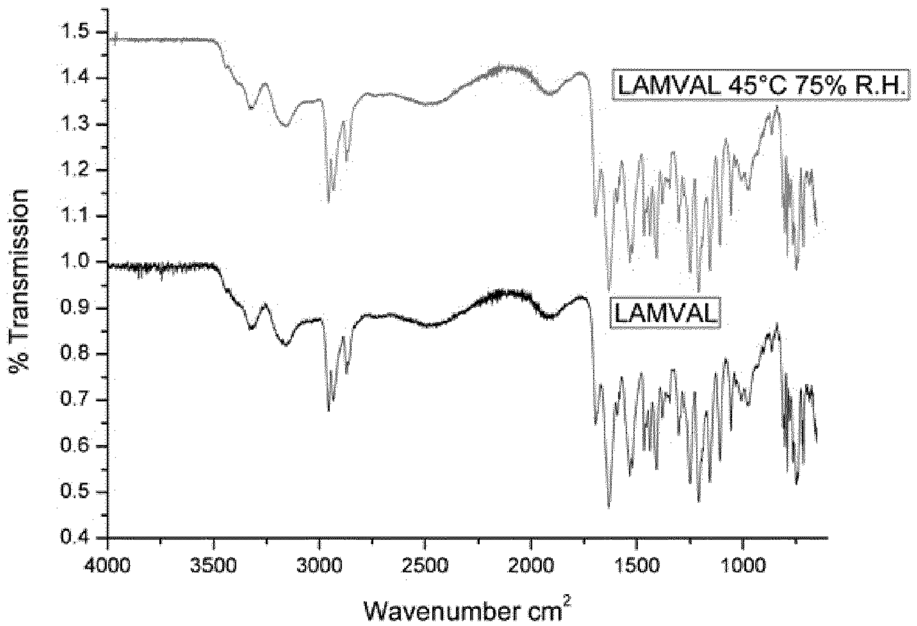
LAMVAL 45°C 75% R.H.
LAMVAL
% Transmission
Wavenumber cm2
1.5
1.4
1.3
1.2
1.1
1.0
0.9
0.8
0.7
0.6
0.5
0.4
4000
3500
3000
2500
2000
1500
1000

**Fig. 7**

7/8

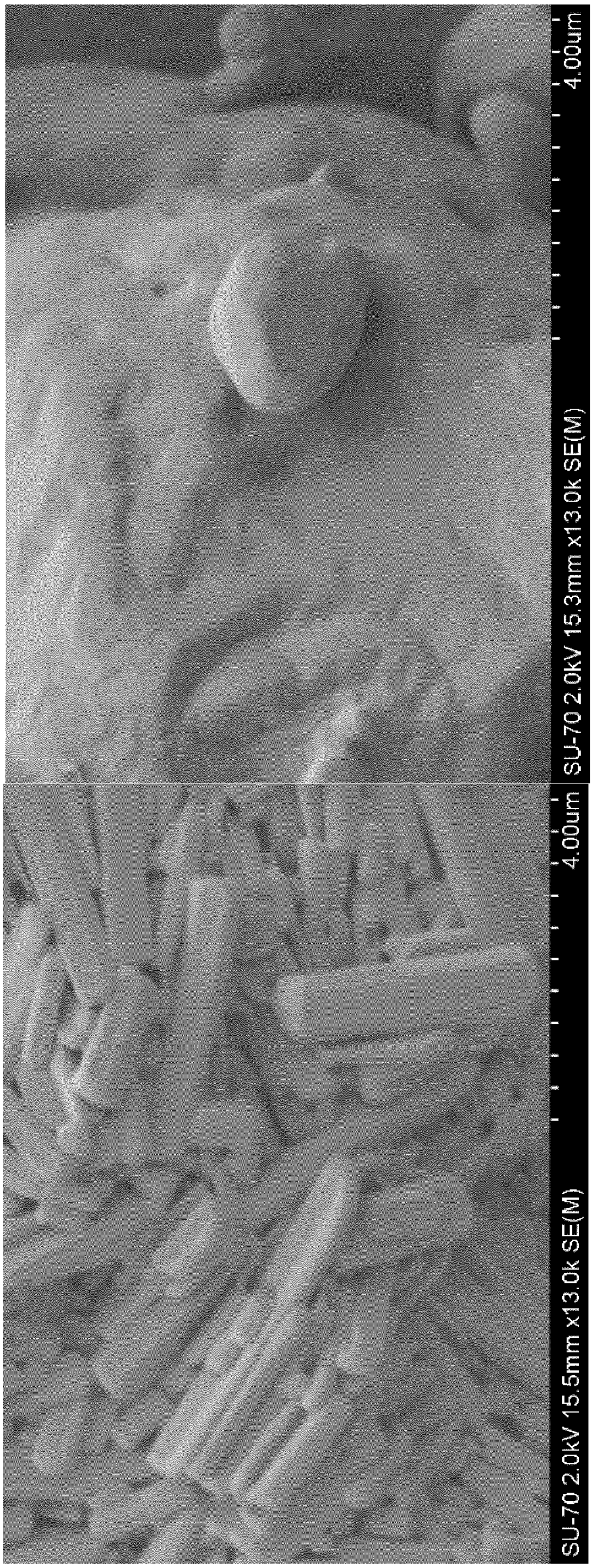
SU-70 2.0kV 15.3mm x13.0k SE(M)
4.00um
SU-70 2.0kV 15.5mm x13.0k SE(M)
4.00um

Fig. 8

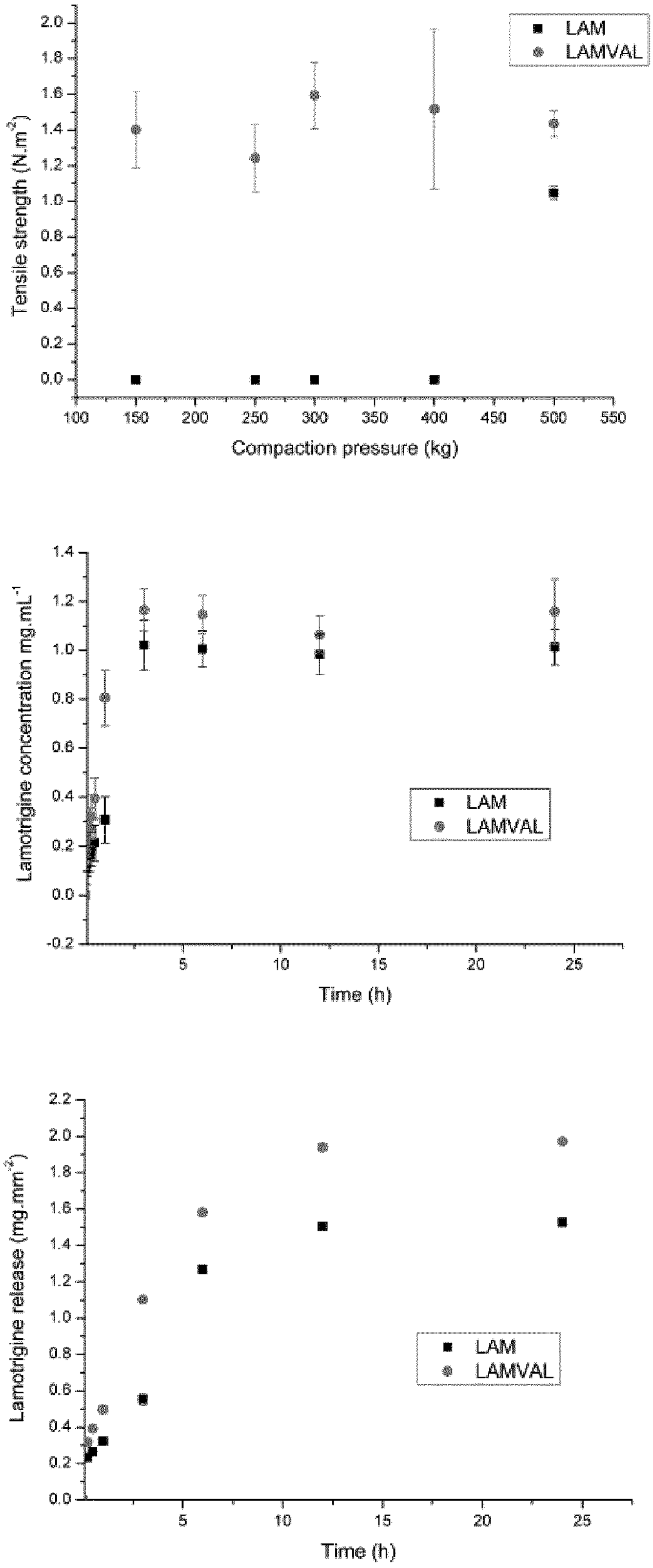
LAM
LAMVAL
Tensile strength (N.m-2)
Compaction pressure (kg)
Lamotrigine concentration mg.mL-1
Time (h)
Lamotrigine release (mg.mm-2)
Time (h)

**Fig. 9**

Europäisches Patentamt
European Patent Office
Office européen des brevets

# EUROPEAN SEARCH REPORT

Application Number
EP 19 16 6734

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | THIPPARABOINA R. ET AL.: "Ionic, neutral, and hybrid acid-base crystalline adducts of lamotrigine with improved pharmaceutical performance", CRYSTAL GROWTH AND DESIGN, vol. 15, 2015, pages 5816-5826, XP002793972, * figures 9,10 * | 1-7,9-15 | INV. A61P25/08 A61K31/19 A61K31/53 A61K45/06 C07D253/02 A61K9/14 |
| X | WO 2009/061513 A1 (THAR PHARMACEUTICALS [US]; HANNA MAZEN [GB] ET AL.) 14 May 2009 (2009-05-14) * figures; examples 8,9,10 * | 1-6,9-15 | |
| X | CHENEY M.L. ET AL.: "Effects of crystal form on solubility and pharmacokinetics: a crystal engineering case study of lamotrigine", CRYSTAL GROWTH AND DESIGN, vol. 10, no. 1, 2010, pages 394-405, XP002793973, * page 397, left-hand column; figures 1,11,12,13; table 3 * | 1-6,9-15 | TECHNICAL FIELDS SEARCHED (IPC) |
| A | CN 103 360 331 B (UNIV TIANJIN) 15 April 2015 (2015-04-15) * the whole document * | 1-15 | A61P A61K C07D |

The present search report has been drawn up for all claims

1

| Place of search | Date of completion of the search | Examiner |
|---|---|---|
| Munich | 3 September 2019 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 16 6734

This annex lists the patent family members relating to the patent documents cited in the above-mentioned European search report. The members are as contained in the European Patent Office EDP file on
The European Patent Office is in no way liable for these particulars which are merely given for the purpose of information.

03-09-2019

| Patent document cited in search report | Publication date | Patent family member(s) | Publication date |
|---|---|---|---|
| WO 2009061513 A1 | 14-05-2009 | US 2009176787 A1<br>WO 2009061513 A1 | 09-07-2009<br>14-05-2009 |
| CN 103360331 B | 15-04-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **P. STAHL.** Handbook of Pharmaceutical Salts: Properties, Selection and Use. VCHA/WNey-VCH, 2002
- **S.M. BERGE.** Pharmaceutical Salts. *Journal of Pharmaceutical Sciences,* 1977, vol. 66, 1-19
- **S. BYRN.** *Pharmaceutical Research,* 1995, vol. 12 (7), 954-954
- **R. LIU.** Water- Insoluble Drug Formulation. CRC Press, 553